# EUROPEAN PATENT SPECIFICATION

(11) **EP 1 487 506 B1**
(45) Date of publication and mention of the grant of the patent: **04.04.2007**
(21) Application number: 03704190.2
(22) Date of filing: 15.01.2003
(51) Int. Cl.: A61L 15/28, A61L 26/00

(54) **PREPARATION FOR WOUND HEALING AND PREVENTION OF BANDAGE ADHESION TO THE WOUND**
ZUBEREITUNG FÜR DIE WUNDHEILUNG UND ZUR VERHINDERUNG DES ANHAFTENS VON VERBÄNDEN AN DIE WUNDE
PREPARATION FAVORISANT LA CICATRISATION ET EMPECHANT L'ADHESION DU PANSEMENT A LA PLAIE

(30) Priority: 18.01.2002 CZ 200212746 U
(43) Date of publication of application: 22.12.2004
(73) Proprietor: CPN Spol. S R.O., 561 02 Dolni Dobrouc (CZ)
(72) Inventor: VELEBNY, Vladimir, 56201 Usti nad Orlici (CZ); SOBOTKA, Lubos, 500 02 Kralove Hradec (CZ); PAVEK, Stanislav, 565 53 Sloupnice (CZ); RUZICKOVA, Jana, 767 01 Kromeriz (CZ)
(74) Representative: Kania, Frantisek
(86) International application number: PCT/CZ2003/000003
(87) International publication number: WO 2003/059404

(56) References cited:
- WO-A-97/02845
- US-A- 5 442 053
- DATABASE WPI Section Ch, Week 199910 Derwent Publications Ltd., London, GB; Class A96, AN 1999-114793 XP002245695 & JP 10 338638 A (TOA YAKUHIN KK), 22 December 1998 (1998-12-22)

## Description

### Technical Field

The present invention relates to a preparation based on a pharmacologically acceptable salt of hyaluronic acid which is applied to the wound and it is able to prevent adhesion of the bandage to the wound and at the same time to speed up the process of healing.

### Background Art

The present state of healing of acute and chronic complicated wounds is based on the use of a whole range of different materials and techniques. Of course the ideal solution is to clean the wound and then take care of it by a surgical treatment (suture, skin auto-grafting, etc.). However said method of treatment is possible only for a very small number of skin defects and it applies to surgical wounds and acute non-infected wounds.

Most of the large acute wounds are infected and they contain necrotic parts. The bacterial contamination can be supposed for chronic wounds and practically in all cases there are some necroses. These large subacute and chronic wounds are very difficult to treat.

At present the following systems described below are used for healing of large or chronic wounds.

One of the systems is a repeated re-bandage with moisten gauzes which assure a permanent damp environment for wound healing. The gauze is moistened by a physiological solution or a solution containing an antiseptic (Betadine, chloramine, rivanol, etc.). Said method is difficult since it requires a frequent re-bandage every 4 to 6 hours. This method also does not lead to the complete sterilization of the wound and it does not prevent a maceration of skin in the wound surroundings.

Since the bandages which are not moistened regularly very often stick to the wound, they are often saturated by vaseline, it is the socalled greasy gauze. The vaseline is often combined with iodine so that the bandage has antimicrobial properties. A disadvantage of such use is that the vaseline closes the wound and all the necrotic parts and infection are cumulated under the bandage. Problems also arise with removing of vaseline from deeper wounds during re-bandaging. For this reason this method is used only for superficial wounds.

Smaller deep wounds are often covered by a plastic bandage which maintains a moist environment in the wound. This bandage comprises cellulose derivatives and sometimes also contains a seaweed extract (alginate). Such bandage meets mainly the covering function since it does not have disinfection effects on the wound and the bandage itself does not contribute to the elimination of the necrotic tissue. There are very complicated and expensive systems available on the market which use for example collagenase or papaine in the ointment or cream base for the elimination of the necrotic tissue. Tissue secretion is removed mainly by preparations containing active coal.

If the necrotic parts are removed and the wound is disinfected, then preparations providing a hydrated environment which should be beneficial for the wound healing are used. This is due mainly to the use of alginate, a polysaccharide, which is produced by seaweeds. Some companies use carboxymethylcellulose for ensuring the hydration of the wound environment, which is essential for its healing. A disadvantage of this system is that it requires cleaning of the wound in a separate step prior to the application of the substances enhancing the wound healing and moreover, it is not able to keep the wound sterile and prevent the later development of infection without further support.

Different growth factors and the sodium salt of hyaluronic acid are used in some preparations for enhancing the healing effects. A disadvantage of the system in this case is that it also requires cleaning of the wound in a separate step prior to the application of substances enhancing the wound healing. Moreover said preparations are not able to keep the wound sterile without further support and prevent the infection development.

A combination of collagen and a chemically modified cellulose is also used for the therapy of chronic wounds. If this system is not completed by other substances it is not able to ensure wound disinfection, removing of secretion, etc. on its own.

Furthermore, some authors recommend the use of oxygenotherapy in hyperbaric chambers for healing of chronic wounds (especially for diabetic patients). However this therapy is very demanding concerning the apparatus.

A vacuum-therapy is a kind of treatment in which porous elastic sponge is applied to the wound and the wound is covered by an impermeable sheet. The air is then exhausted from the wound and the incurred underpressure should ensure cleaning of the wound and secretion exhausting. The sponge is exchanged in regular intervals. This is very apparatus demanding and it is suitable only for highly specialized workplaces, and moreover, it is limited for selected diagnoses only.

WO 97/02845 A1 discloses a film dressing for topical use in the treatment of wounds comprising sodium hyaluronate. The film may also comprise antibacterial agents.

### Disclosure of Invention

The aim of the invention is to create a preparation, the application of which to the wound would provide an environment that prevents wound infections and disinfects the wound at the same time. Furthermore, it would exhaust the secretion from the wound and thus prevent the maceration of the wound and its surroundings and maintain a good hydration in the wound and the presence of tissue mediators and enzymes. The preparation should also ensure an ideal environment needed for the formation of granulation tissue and other regeneration processes in the wound. It should prevent the bandage adhesion to the wound, protect the wound surroundings and enable monitoring of possible wound changes (especially development of bleeding).

The disadvantages stated in the background of the invention and the aims laid out above are solved by the preparation for wound healing and prevention of bandage adhesion to the wound according to the invention. The subject-matter of the invention is a preparation containing physiologically acceptable salt of hyaluronic acid having the molecular weight in the range from 200000 to 2500000 in gel or solution together with iodine and potassium iodide.

A preferred physiologically acceptable salt of hyaluronic acid is selected from a group containing sodium salt, potassium salt, lithium salt, calcium salt, magnesium salt, zinc salt, cobalt salt, manganese salt or a combination thereof. The preparation according to the invention is preferably in the form of viscous aqueous solution or gel.

The preparation according to the invention preferably contains a physiologically acceptable salt of hyaluronic acid in the concentration from 0,05 to 10 % by weight, iodine in the concentration from 0,05 to 2,5 % by weight and potassium iodide in the concentration from 0,05 to 5 % by weight as substances with antiseptic properties acting bacteriostatically and fungistatically.

A preferred embodiment of the invention is a preparation containing a physiologically acceptable salt of hyaluronic acid in the concentration from 0,05 to 10,0 % by weight, iodine in the concentration from 0,075 to 1 % by weight and potassium iodide in the concentration from 0,075 to 1 % by weight.

The preparation according to the invention is prepared by dissolving the above mentioned substances in sterile water.

The preparation for wound healing and prevention of bandage adhesion to the wound is applied either directly to the wound or is spread in the needed amount on that side of bandage which is then placed on the wound.

A combination of suitable salts of hyaluronic acid with iodine and potassium iodide is itself able to satisfy the above mentioned conditions. Salts of hyaluronic acid belong to the most hydrophilic molecules in nature. The preparation ensures the secretion of tissue fluid after its application on the gauze and the wound and also a constantly damp environment. In a combination with iodine and potassium iodide it disinfects the wound for a short time which provides a clean environment in the wound. The salts of hyaluronic acid have also a strong healing effect, they act very positively during all phases of the healing process. This all has a positive effect on the formation of the granulation tissue and the following epithelisation and thereby the healing of the wound. The advantage is also the possibility of bandage monitoring and the fact that only the wound itself is hydrated and the skin around the wound is intact.

The preparation according to the invention activates keratinocytes to produce cytokines in contrary to iodine and potassium iodide separately (an iodine complex) and hyaluronan separately. The cytokines produced are the activators and chemoatractants for different cells of white line which shows up in a speeded wound cleaning and a preparation of the wound surface for the formation of the granulation tissue. Furthermore, they activate keratinocytes which allows the ingrowth of the wound. The above mentioned unexpected effects are not exhibited by either one of the three components of the preparation according to the invention if applied separately. Iodine in combination with another oligomer or polymer substances is used in some preparations (e.g. Betadine). In our case, it is not possible to use the combination of hyaluronan as a polymer substance and iodine directly since it is not possible to reach the required concentration of iodine in solution. For this reason potassium iodide is added forming the iodine complex. The iodine complex has the requested solubility in water as well as the combination of iodine and potassium iodide is more acceptable for the cells than the iodine alone.

### Examples of the invention

### Example 1

0,1 g of iodine is dissolved in the solution of 0,15 g of potassium iodide in 50 ml of sterile water for injections. Furthermore, 1,5 g of sodium hyaluronate having the molecular weight 1 000 000 is dissolved in 50 ml of sterile water for injections. The solutions are prepared separately and they are separately sterilized. They are mixed together under sterile conditions after sterilization. The highly viscous solution that is produced can be applied directly to the wound which is afterwards covered by the bandage or it can be applied to the bandage which is afterwards placed on the wound.

### Example 2

1,0 g of iodine is dissolved in the solution of 1,5 g of potassium iodide in 50 ml of sterile water for injections. Furthermore, 1,5 g of sodium hyaluronate having the molecular weight 1 500 000 is dissolved in 50 ml of sterile water for injections. The solutions are prepared separately and they are separately sterilized. They are mixed together under sterile conditions after sterilization. The highly viscous solution which is produced can be applied directly to the wound which is covered afterwards by the bandage or it can be applied to the bandage, which is afterwards placed on the wound.

### Example 3

0,5 g of iodine is dissolved in the solution of 0,75 g of potassium iodide in 50 ml of sterile water for injections. The gel of potassium hyaluronate having the molecular weight 1 500 000 is produced by mixing of 2 g of hyaluronan with 50 ml of water for injections in a separate flask. The solution and the gel are prepared separately and they are also separately sterilized. They are mixed together under sterile conditions after sterilization. It is possible to apply the produced gel in a thin layer directly to the wound which is afterwards covered by the bandage.

## Claims

1. Preparation for wound healing and prevention of adhesion to the wound **characterized by** the content of a physiologically acceptable salt of hyaluronic acid having the molecular weight from 200 000 to 2 500 000, iodine and potassium iodide.

2. Preparation of claim 1 **characterized in that** said physiologically acceptable salt of hyaluronic acid is selected from a group containing sodium salt, potassium salt, lithium salt, calcium salt, magnesium salt, zinc salt, cobalt salt and manganese salt or a combination thereof.

3. Preparation of claim 1 **characterized in that** the concentration of the physiologically acceptable salt of hyaluronic acid is in the range from 0,05 to 10,0 % by weight, the concentration of iodine is in the range from 0,05 to 2,5 % by weight and the concentration of potassium iodide is in the range from 0,05 to 5 % by weight.

4. Preparation of claim 3 **characterized in that** the concentration of the physiologically acceptable salt of hyaluronic acid is in the range from 0,05 to 10,0 % by weight, the concentration of iodine is in the range from 0,075 to 1 % by weight and the concentration of potassium iodide is in the range from 0,075 to 1 % by weight.

5. Preparation according to claim 1 **characterized by** being in the form of a sterile aqueous solution or a gel.

## Patentansprüche

1. Zubereitung fiir Wundenbehandlung und Prävention einer Adhäsion an der Wunde **gekennzeichnet durch** den Inhalt des physiologisch zulässigen Salzes der hyaluronischen Säure mit dem Molekulargewicht von 200 000 bis 2 500 000, des Jods und des Kaliumjodids.

2. Zubereitung nach Anspruch 1, **dadurch gekennzeichnet, daß** das physiologisch zulässige Salz der hyaluronischen Säure aus der das Natronsalz, Kaliumsalz, Lithiumsalz, Kalziumsalz, Magnesiumsalz, Zinksalz, Kobaltsalz und Mangansalz oder dessen Kombination beinhaltenden Gruppe gewählt wird.

3. Zubereitung nach Anspruch 1, **dadurch gekennzeichnet, daß** die Konzentration des physiologisch zulässigen Salzes der hyaluronischen Säure im Bereich von 0,05 bis 10 Gewichtsprozent, die Konzentration des Jods im Bereich von 0,05 bis 2,5 Gewichtsprozent und die Konzentration des Kaliumjodids im Bereich von 0,05 bis 5 Gewichtsprozent ist.

4. Zubereitung nach Anspruch 3 **dadurch gekennzeichnet, daß** die Konzentration des physiologisch zulässigen Salzes der hyaluronischen Säure im Bereich von 0,05 bis 10 Gewichtsprozent, die Konzentration des Jods im Bereich von 0,075 bis 1 Gewichtsprozent und die Konzentration des Kaliumjodids im Bereich von 0,075 bis 1 Gewichtsprozent ist.

5. Zubereitung nach Anspruch 1, **dadurch gekennzeichnet, daß** diese in der Form einer sterilen wäßrigen Lösung oder eines Gels ist.

## Revendications

1. Préparation pour guérir une blessure et pour une prévention d'une adhérence à la blessure, **caracterisé en ce que** la contenu d'un sel d'acid hyaluronique physiologiquement addmissible a le poids moléculaire de 200 000 à 2 500 000, iode et iodure de potassium.

2. Préparation selon la révendication 1, **caracterisé en ce que** le sel d' acid hyaluronique physiologiquement addmissible est choisi du groupe comprenant un sel de sodium, un sel de potassium, un sel de lithium, un sel de calcium, un sel de magnésium, un sel de zinc, un sel de cobalt et un sel de manganése ou la combinaison à eux.

3. Préparation selon la révendication 1, **caracterisé en ce que** la concentration du sel d'acid hyaluronique physiologiquement addmissible est dans l'étendu de 0,05 à 10,0 % de poids, la concentration de l'iode est dans l'étendu de 0,05 à 2,5 % de poids et la concentration de l'iodure de potassium est dans l'étendu de 0,05 à 5 % de poids.

4. Préparation selon la révendication 3, **caracterisé en ce que** la concentration du sel d'acid hyaluronique physiologiquement addmissible est dans l'étendu de 0,05 à 10,0 % de poids, la concentration de l'iode est dans l'étendu de 0,075 à 1 % de poids et la concentration de l'iodure de potassium est dans l'étendu de 0,075 à 1 % de poids

5. Préparation selon la révendication 1, **caracterisé en ce qu'**elle est en forme d'une solution aqueuse stérile ou du gel.
